Europäisches Patentamt

(19) European Patent Office

Office européen des brevets

(11) Publication number: **0 068 755**
**B1**

(12) **EUROPEAN PATENT SPECIFICATION**

(45) Date of publication of patent specification: 07.05.86

(21) Application number: 82303182.8

(22) Date of filing: 18.06.82

(51) Int. Cl.⁴: **B 01 J 29/28**, C 07 C 2/66,
C 07 C 6/12, C 07 C 15/02

(54) Treatment of zeolite catalysts to enhance para-selectivity.

(30) Priority: 26.06.81 US 277484
26.06.81 US 277485
26.06.81 US 277486

(43) Date of publication of application:
05.01.83 Bulletin 83/01

(45) Publication of the grant of the patent:
07.05.86 Bulletin 86/19

(84) Designated Contracting States:
BE DE FR GB IT NL

(56) References cited:
EP-A-0 012 570
FR-A-2 036 709
US-A-3 178 365
US-A-3 853 743
US-A-4 049 573
US-A-4 098 837
US-A-4 250 345

(73) Proprietor: MOBIL OIL CORPORATION
150 East 42nd Street
New York New York 10017 (US)

(72) Inventor: Kaeding, Warren William
700 Mountain Avenue
Westfield New Jersey (US)

(74) Representative: Cooper, John Anthony et al
CARPMAELS & RANSFORD 43 Bloomsbury
Square
London WC1A 2RA (GB)

Courier Press, Leamington Spa, England.

## Description

The present invention relates to the preparation and use of modified zeolite catalyst compositions which are especially suitable for the conversion of substituted aromatic hydrocarbons to provide product mixtures enriched in the para-(or 1,4-) dialkyl substituted benzene isomer.

Production of dialkyl substituted benzene compounds via disproportionation, alkylation and/or transalkylation of aromatic hydrocarbons is an important step in a number of commercial chemical manufacturing processes. Such reactions can be carried out over a variety of catalyst materials. Alkylation of aromatic hydrocarbons utilizing crystalline aluminosilicate catalysts has, for example, been described. U.S.—A—2,904,697 refers to alkylation of aromatic hydrocarbons with an olefin in the presence of a crystalline metallic aluminosilicate having uniform openings of about 6 to 15 Angstrom units. U.S.—A—3,251,897 describes alkylation of aromatic hydrocarbons in the presence of X or Y-type zeolites, specifically such zeolites wherein the cation is rare earth and/or hydrogen. U.S.—A—4,086,287 discloses the use of ZSM-5 type zeolites as catalysts for the alkylation of aromatic hydrocarbons such as toluene. U.S.—A—3,751,504 and 3,751,506 describe vapor phase alkylation of aromatic hydrocarbons with olefins, e.g., benzene with ethylene, in the presence of a ZSM-5 type zeolite catalyst.

The disproportionation of aromatic hydrocarbons in the presence of zeolite catalysts has been described by Grandio et al in the *Oil and Gas Journal*, Vol. 69, No. 48 (1971), U.S.—A—3,126,422; 3,413,374; 3,598,878; 3,598,879 and 3,607,961 show vapor-phase disproportionation of toluene over various catalysts.

In many of these prior art processes, the dialkylbenzene product produced contains more of the 1,3-isomer than either of the other two isomers. In the conventional methylation of toluene to form xylene, the product has the equilibrium composition of approximately 24 percent of 1,4-, 54 percent of 1,3- and 22 percent of 1,2-isomer. Of the dialkylbenzene isomers, 1,3-dialkylbenzene is often the least desired product, with 1,2- and 1,4-dialkylbenzene being the more useful products. 1,4-Dimethylbenzene, for example, is of particular value, being useful in the manufacture of terephthalic acid which is an intermediate in the manufacture of synthetic fibers such as "Dacron". Furthermore, 1,4-methylethylbenzene, i.e., para-ethyltoluene (PET), is useful for subsequent conversion to para-methylstyrene, and for this purpose ethyltoluene products containing as much as 97% of the para isomer are required.

Mixtures of dialkylbenzene isomers, either alone or in further admixture with ethylbenzene, have previously been separated by expensive superfractionation and multistage refrigeration steps. Such processes, as will be realized, involve high operation costs and have a limited yield. Alternatively, various modified zeolite catalysts have been developed to alkylate or disproportionate toluene with a greater or lesser degree of selectivity to 1,4-dialkylbenzene isomers. Hence, U.S.—A—3,972,832, 4,034,053, 4,128,592, and 4,137,195 disclose particular zeolite catalysts which have been treated with compounds of phosphorus and/or magnesium to increase para-selectivity of the catalysts. Para-selective boron-containing zeolites are shown in U.S.—A—4,067,920 and para-selective antimony-containing zeolites in U.S.—A—3,979,472. Similarly, U.S.—A—3,965,208 and 4,117,026 disclose other modified zeolites useful for shape selective reactions.

Notwithstanding the existence of such modified zeolite catalysts having para-selective properties, there is a continuing need to develop additional types of catalytic materials which are highly para-selective when used for the conversion of aromatic compounds to dialkylbenzene products. Accordingly, it is an object of the present invention to provide modified zeolite catalyst compositions which promote the conversion of aromatics to produce mixtures containing an exceptionally high percentage, e.g., 97% by weight or more, for alkylation of toluene, of para-dialkylbenzene isomer.

It is a further object of the present invention to provide such highly para-selective catalysts without necessarily resorting to expensive and/or time consuming catalyst selectivation techniques such as steaming and/or precoking after each instance of catalyst regeneration.

According to the present invention a process for modifying zeolite catalysts to render such catalysts highly para-selective for the conversion of aromatic compounds dialkyl substituted benzene compounds, the zeolite being free of organic cations and having a silica to alumina mole ratio of at least 12 and a constraint index within the approximate range of 1 to 12, comprises treatment with $CO_2$, $NO_2$, $NH_3$, $SO_2$ or $H_2S$ at a temperature of 50 to 500°C for 0.1 to 25 hours followed by calcination of the treated catalyst at a temperature of 300 to 600°C.

The preferred zeolites are ZSM-5, ZSM-11, ZSM-12, ZSM-23, ZSM-35, ZSM-38 and ZSM-48 defined respectively by the X-ray data set forth in U.S.—A—3,702,886, 3,709,979, 3,832,449, 4,076,842, 4,016,245, and 4,046,859, and in EP—A—15132.

In all of the foregoing zeolites, the original cations can be subsequently replaced, at least in part, by calcination and/or ion exchange with another cation. Thus, the original cations can be exchanged into a hydrogen or hydrogen ion precursor form or a form in which the original cations have been replaced by a metal of, for example, Groups II through VIII of the Periodic Table. Thus, it is contemplated to exchange the original cations with ammonium ions or with hydronium ions. Catalytically active forms of these would include, in particular, hydrogen, rare earth metals, aluminum, manganese and other metals of Groups II and VIII of the Periodic Table.

The specific zeolites described, when prepared in the presence of organic cations, are substantially catalytically inactive, possibly because the intra-crystalline free space is occupied by organic cations from the forming solution. They may be activated by heating in an inert atmosphere at 540°C for one hour, for example followed by base exchange with ammonium salts followed by calcination in 540°C in air. The presence of organic cations in the forming solution may not be absolutely essential to the formation of these zeolites but does appear to favor it. More generally, it is desirable to activate this type catalyst by base exchange with ammonium salts followed by calcination in air at about 540°C for from about 15 minutes to about 24 hours. Zeolites treated according to the present invention are free of organic cations.

In practicing any given desired hydrocarbon conversion process, including those of the present invention, it may be useful to incorporate the zeolite into a matrix comprising material resistant to the temperature and other conditions employed in the process. Such a matrix is useful as a binder and imparts greater resistance to the catalyst for the severe temperature, pressure and reactant feed stream velocity conditions encountered in many processes. Suitable matrix materials are described in our EP—A—1695. The process according to the invention may be carried out either before or after such incorporation.

When the catalyst compositions of the type hereinbefore described are to be used for the conversion of aromatic compounds, the para-selective properties of such catalysts can preferably be enhanced in known manner by the treatment of such catalysts with difficultly reducible oxides of a number of elements prior to treatment with carbon dioxide in accordance with the present invention. Most commonly such catalysts are treated with phosphorus and/or magnesium compounds in the manner described in U.S.—A—3,894,104, 4,049,573, 4,086,287 and 4,128,592.

Phosphorus, for example, can be incorporated into such zeolites in the form of phosphorus oxide in an amount of from about 0.25% to about 25% by weight of the catalyst composition. Such incorporation can be readily effected by contacting the zeolite composite with a solution of an appropriate phosphorus compound, followed by drying and calcining to convert the phosphorus compound to its oxide form.

As noted, magnesium is another material commonly incorporated onto the zeolite composites of the present invention thereby to enhance their para-selectivity. Magnesium generally is incorporated as magnesium oxide and can be utilized either as the sole modifying agent or in combination with oxides of phosphorus as described hereinbefore or with other materials.

The amount of magnesium oxide incorporated in the zeolite should be at least about 0.25 percent by weight. However, it is preferred that the amount of magnesium oxide in the zeolite be at least about 1 percent by weight, particularly when the same is combined with a binder, e.g., 35 weight percent of alumina. The amount of magnesium oxide can be as high as about 25 percent by weight or more depending on the amount and type binder present. Preferably, the amount of magnesium oxide added to the zeolite is between about 1 and about 15 percent by weight.

In addition to treatment of the zeolite composites with phosphorus and/or magnesium as hereinbefore described in detail, such zeolites may also be treated with a variety of other oxide materials to enhance para-selectivity. Such oxide materials include oxides of boron (U.S. 4,067,920); antimony (U.S. 3,979,472); and beryllium, Group VIIA metals, alkaline earth metals, Group IB metals, Group IVB metals, Group VIA metals, Group IA elements, cadmium, iron and/or cobalt, Group IIIB metals, Group IVA metals, Group VA metals, and Group IIIA elements (described in EP—A—51346, 50907, 37630, 37168, 38116, 36707, 36249, 35836, 40463, 40900, 39536 and 34918).

Treatment of the zeolite catalysts with any of the foregoing oxide materials to enhance para-selectivity will generally occur before such catalysts are treated in accordance with the present invention in order to provide even greater enhancement of the para-selective properties of such catalysts. Additional catalyst modifying procedures which may also optionally be employed to enhance catalyst para-selectivity include precoking and steaming, or combinations thereof.

Steaming entails contact of the zeolite with an atmosphere containing from about 5 to about 100 percent steam at a temperature of from about 250°C to about 1000°C for a period of between about 15 minutes and about 100 hours and under pressures ranging from sub-atmospheric to several hundred atmospheres. Preferably, steam treatment is effected at a temperature of between about 400°C and about 700°C for a period of between about 1 and about 24 hours.

Precoking of the catalyst serves to deposit a coating of between about 2 and about 75, and preferably between about 15 and about 75, weight percent of coke thereon to enhance catalyst selectivity. Precoking can be accomplished by contacting the catalyst with a hydrocarbon charge, e.g., toluene, under high severity conditions or alternatively at a reduced hydrogen to hydrocarbon relative concentration, i.e., 0 to 1 mole ratio of hydrogen to hydrocarbon, for a sufficient time to deposit the desired amount of coke thereon.

The zeolite catalyst composites described above, whether or not modified by treatment with phosphorus and/or magnesium oxides or other oxide materials, or modified by steaming or precoking techniques, are treated in accordance with the present invention to provide even further enhancement of the para-selective properties of the catalyst, under conditions which serve to enhance para-selectivity of the catalysts so treated. Catalyst treating conditions will vary with the concentration of the treating agent employed. However, in general such para-selectivity enhancing catalyst treating conditions will include contact of the catalyst with a medium, preferably anhydrous, containing the $CO_2$, $NO_2$, $NH_3$, $SO_2$ or $H_2S$, at temperatures of 50°C to 500°C for a period of 0.1 to 25 hours, followed by calcination of the treated catalyst at temperatures of from about 300°C to 600°C.

The medium containing the treating agent will generally be gaseous and will contain from about 25% to 100% by volume of the agent. Catalyst treatment operations may take place within the aromatics conversion reactor itself or may take place in a separate catalyst treatment vessel.

The treating medium is preferably passed over the catalyst at the rate of 20 to 100 cc/minute/gram catalyst at a temperature of 50°C to 500°C for a period of 0.25 to 3 hours.

Treatment of catalysts in accordance with the present invention can permit elimination of the need for steaming and/or precoking procedures in order to reach given levels of para-selectivity, particularly after regeneration of such catalysts with air or other oxygen-containing gas.

Alkylation of aromatic compounds in the presence of the treated catalyst is effected by contact of the aromatic with an alkylating agent. A particularly preferred embodiment involves the alkylation of toluene wherein the alkylating agents employed comprise methanol or other well known methylating agents or ethylene. The reaction is carried out at a temperature of between 250°C and 750°C, preferably between 300°C and 650°C. At higher temperatures, the zeolites of high silica/alumina ratio are preferred. For example, ZSM-5 having a $SiO_2/Al_2O_3$ ratio of 30 and upwards is exceptionally stable at high temperatures. The reaction generally takes place at atmospheric pressure, but pressures within the approximate range of $10^5$ $N/m^2$ to $10^7$ $N/m^2$ (1—100 atmospheres) may be employed.

Some non-limiting examples of suitable alkylating agents would include olefins such as, for example, ethylene, propylene, butene, decene and dodecene, as well as formaldehyde, alkyl halides and alcohols, the alkyl portion thereof having from 1 to 16 carbon atoms. Numerous other aliphatic compounds having at least one reactive alkyl radical may be utilized as alkylating agents.

Aromatic compounds which may be selectively alkylated as described herein would include any alkylatable aromatic hydrocarbon such as, for example, benzene, ethylbenzene, toluene, dimethylbenzene, diethylbenzene, methylethylbenzene, propylbenzene, isopropylbenzene, isopropylmethylbenzene, or substantially any mono- or di-substituted benzenes which are alkylatable in the 4-position of the aromatic ring.

The molar ratio of alkylating agent to aromatic compound is generally between 0.05 and 5. For instance, when methanol is employed as the methylating agent and toluene is the aromatic, a suitable molar ratio of methanol to toluene has been found to be approximately 0.1 to 1.0 mole of methanol per mole of toluene. When ethylene is employed as the alkylating agent and toluene is the aromatic, a suitable molar ratio of ethylene to toluene is approximately 0.05 to 2.5 moles of ethylene per mole of toluene.

Alkylation is suitably accomplished utilizing a feed weight hourly space velocity (WHSV) of between 1 and 1000, and preferably between 1 and 200. The reaction product, consisting predominantly of the 1,4-dialkyl isomer, e.g., 1,4-dimethylbenzene, 1-ethyl-4-methylbenzene, etc., or a mixture of the 1,4- and 1,3- isomers together with comparatively smaller amounts of 1,2-dialkylbenzene isomer, may be separated by any suitable means. Such means may include, for example, passing the reaction product stream through a water condenser and subsequently passing the organic phase through a column in which chromatographic separation of the aromatic isomers is accomplished. Alkylation using the treated catalysts of the present invention can provide product mixtures containing at least 90% or even 95% or more by weight of the para-dialkylbenzene isomer.

When transalkylation is to be accomplished, transalkylating agents are alkyl or polyalkyl aromatic hydrocarbons wherein alkyl may be composed of from 1 to about 5 carbon atoms, such as, for example, toluene, xylene, trimethylbenzene, triethylbenzene, dimethylethylbenzene, ethylbenzene, diethylbenzene, ethyltoluene, and so forth.

Another process embodiment of this invention relates to the selective disproportionation of alkylated aromatic compounds to produce dialkylbenzenes wherein the yield of 1,4-dialkyl isomer is in excess of the normal equilibrium concentration. In this context, it should be noted that disproportionation is a special case of transalkylation in which the alkylatable hydrocarbon and the transalkylating agent are the same compound, for example when toluene serves as the donor and acceptor of a transferred methyl group to produce benzene and xylene.

The transalkylation and disproportionation reactions are carried out by contacting the reactants with the above described modified zeolite catalyst at a temperature between 250°C and 750°C at a pressure between atmospheric ($10^5$ $N/m^2$) and 100 atmospheres ($10^7$ $N/m^2$). The reactant feed WHSV will normally fall within the range of 0.1 to 50. Preferred alkylated aromatic compounds suitable for utilization in the disproportionation embodiment comprise toluene, ethylbenzene propylbenzene or substantially any mono-substituted alkylbenzene. These aromatic compounds are selectively converted to, respectively, 1,4-dimethylbenzene, 1,4-diethylbenzene, 1,4-dipropylbenzene, or other 1,4-dialkylbenzene, as appropriate, with benzene being a primary side product in each instance. The product is recovered from the reactor effluent by conventional means, such as distillation, to remove the desired products of benzene and dialkylbenzene, and any unreacted aromatic component is recycled for further reaction.

The hydrocarbon conversion processes described herein may be carried out as a batch type, semi-continuous or continuous operation utilizing a fixed or moving bed catalyst system. The catalyst after use in a moving bed reactor is conducted to a regeneration zone wherein coke is burned from the catalyst in an oxygen-containing atmosphere, e.g. air, at an elevated temperature, after which the regenerated catalyst is recycled to the conversion zone for further contact with the charge stock. In a fixed bed reactor, regeneration is carried out in a conventional manner where an inert gas containing a small amount of oxygen (0.5—2%) is used to burn the coke in a controlled manner so as to limit the temperature to a maximum of around 500°—550°C.

The following examples illustrate certain specific embodiments of the hereindisclosed invention but should not be construed as limiting the scope of the invention.

Example I (comparative)

HZSM-5 zeolite (60.5 grams) having a crystal size of about 2 microns in the form of 1/16 inch diameter extrudate with a 35 weight percent alumina binder was steamed at 600°C for 1 hour. The steamed material was then impregnated with a solution of 38.7 grams of diammonium acid phosphate in 100 ml. of water, dried and calcined at 500°C for about 2 hours in an open dish. The resulting product was cooled and impregnated with a solution of 195 grams of magnesium acetate tetrahydrate in 133 ml. of water, dried and calcined at 500°C for about 16 hours. The final catalyst contained 4.93 weight percent magnesium, present as the oxide, and 3.48 weight percent phosphorus, present as the oxide and served as base for evaluation of catalysts treated in accordance with the invention.

Example II

A procedure was established to evaluate various test catalysts, including the base catalyst of Example I, for their performance in catalyzing para-selective aromatic conversion reactions. In accordance with the procedure, 2.2 grams of the test catalyst, 14—24 mesh, is centered in a quartz reactor. Low surface area quartz chips are used to position the catalyst and fill void spaces. After calcination with air at 500°C for one hour, the temperature is adjusted to 425°C. Toluene is fed to the reactor at a rate of 8.8 cc/hr. with a WHSV of 3.5. A temperature rise occurs, and temperature is immediately adjusted to 450°C. After 25 minutes on stream at 450°C, the liquid product is collected for a period of 5 minutes, for analysis. A 2 cc gas sample is also taken at this time for analysis at a position just after the water condenser. The temperature is then increased rapidly and successively to 500°C, 550°C and 600°C. In a similar manner, liquid and gaseous samples are taken for analysis at each temperature during the last five minutes of a 30-minute run. This series of tests is used to determine performance for selective toluene disproportionation to produce p-xylene and benzene.

The reactor is then purged with nitrogen (without regeneration) and the temperature adjusted to 375°C. Toluene is fed at a rate of 19.8 cc/hr, WHSV of 7.8, then ethylene is added at 15.6 cc/min., WHSV of 0.5, and the nitrogen purge is stopped. The temperature is rapidly adjusted to 400°C. In a similar manner, gaseous and liquid samples are taken during the last five minutes of a 30-minute run. An additional test run is made at 450°C. This series of tests is used to determine performance for the alkylation of toluene with ethylene to produce p-ethyltoluene.

Using these catalyst evaluation procedures, the base catalyst prepared as in Example I was tested for its performance in the toluene disproportionation and alkylation reactions described. Results for two runs are provided in Table I.

TABLE I

Para-selectivity of Mg/P treated ZSM-5 base catalyst

| Reaction | Selectivity to para-isomer (% by weight) | |
| --- | --- | --- |
| | Run 1 | Run 2 |
| Toluene disproportionation | | |
| 450°C | 69.3 | 68.2 |
| 500°C | 65.9 | 64.3 |
| 550°C | 63.4 | 62.2 |
| 600°C | 57.0 | 60.7 |
| Conversion range: (% by wt.) | 1.1—11.0 | 1.1—12.7 |
| Toluene alkylation w/ethylene | | |
| 400°C | 93.7 | 93.0 |
| 450°C | 92.2 | 92.2 |
| Conversion range: (% by wt.) | 16.5—12.4 | 14.8—13.2 |

The Table I data illustrate that the Mg/P-treated base catalyst sample exhibits good para-selectivity which, unlike conversion, decreases with increase in temperature. In accordance with prior art procedures,

**0 068 755**

such a catalyst can be coke selectivated to increase para-selectivity to levels of about 97% for toluene alkylation.

Example III

To illustrate the catalyst treatment process of the present invention, the base catalyst as prepared in Example I is further treated by contact with carbon dioxide. Prior to conversion testing, anhydrous carbon dioxide is passed over the catalyst in the reactor at 100°C for 15 minutes at the rate of about 50 cc/min/2.2 g catalyst. This is followed by a brief purge and then by calcination in air for 1 hour at 500°C. A similar treatment is given a second catalyst sample except that $CO_2$ contact is at 500°C and lasts for 30 minutes. Catalyst samples so treated are then tested for their disproportionation and alkylation performance in the manner described in Example II. Results of such testing are provided in Table II.

TABLE II
Para-selectivity of Mg/P ZSM-5 catalyst
treated with carbon dioxide

| Treating agent | $CO_2$ | $CO_2$ |
|---|---|---|
| Treating-procedure | 15 min/100°C | 30 min/500°C |
| Run No. | 1 | 2 |
| Tol. disproport. | Selectivity to para isomer, wt % | |
| 450°C | 78.0 | 79.2 |
| 500°C | 78.6 | 79.6 |
| 550°C | 74.0 | 74.7 |
| 600°C | 73.5 | 74.7 |
| Conv. % | 0.8—12.1 | 0.8—11.9 |
| Tol+$C_2H_4$ | | |
| 400°C | 95.8 | 96.2 |
| 450°C | 94.7 | 95.2 |
| Conv. % | 14.6—12.6 | 15.4—12.8 |

The Table II data illustrate the increase in para-selectivity provided by treatment of the base catalyst with $CO_2$. Furthermore, it is evident that the decrease in para-selectivity with increased temperature was reduced significantly by $CO_2$ treatment.

Example IV

The base catalyst as prepared in Example I is further treated by contact with $NO_2$. Prior to conversion testing, anhydrous gaseous nitrogen dioxide is passed over the catalyst in the reactor at 100°C for 15 minutes at the rate of about 45 cc/min/2.2 g catalyst. This is followed by a brief purge and then by calcination in air for 1 hour at 500°C. Catalyst so treated is then tested for its disproportionation and alkylation performance in the manner described in Example II. Results of each testing are provided in Table III.

# 0 068 755

## TABLE III
### Para-selectivity of Mg/P and NO₂ treated ZSM-5 catalyst

| Reaction | Selectivity to para-isomer (% by weight) | Conversion range (% by weight) |
|---|---|---|
| Toluene disproportionation | | 0.6—12.3 |
| 450°C | 84.2 | |
| 500°C | 90.5 | |
| 550°C | 91.6 | |
| 600°C | 89.8 | |
| Toluene alkylation w/ethylene | | 11.5—7.5 |
| 400°C | 98.8 | |
| 450°C | 98.4 | |
| Toluene alkylation w/methanol | | 5.9—10.9 |
| 400°C | 97.5 | |
| 500°C | 96.7 | |
| 600°C | 92.8 | |

The Table III data illustrate the increase in para-selectivity provided by treatment of the base catalyst with $NO_2$. Furthermore, it is evident that the decrease in para-selectivity with increased temperature was reduced significantly by $NO_2$ treatment. Para-selectivity, in fact, generally increased with temperature for toluene disproportionation.

Example V

The base catalyst of Example I was further treated with gaseous ammonia ($NH_3$), and samples were tested for toluene conversion performance in the manner described in Example II. Catalyst treating conditions and conversion results are shown in Table IV.

## TABLE IV
### Para-selectivity of Mg/P ZSM-5 catalyst treated with ammonia

| Nitrogen-based treating agent | $NH_3$ | $NH_3$ |
|---|---|---|
| Treating-procedure | 15 min/100°C | 30 min/500°C |
| Run No. | 1 | 2 |
| Tol. disproport. | Selectivity to para isomer, wt % | |
| 450°C | 81.1 | 82.1 |
| 500°C | 83.7 | 81.4 |
| 550°C | 78.9 | 81.5 |
| 600°C | 76.2 | 79.1 |
| Conv. % Tol+$C_2H_4$ | 0.9—12.7 | 0.7—12.9 |
| 400°C | 96.1 | 96.8 |
| 450°C | 95.2 | 96.2 |
| Conv. % | 16.2—12.2 | 15.6—12.0 |

7

The Table IV data illustrate that catalyst treatment with $NH_3$, like treatment with $NO_2$, serves to enhance para-selectivity of Mg/P modified ZSM-5 catalyst for the toluene conversion reactions involved.

Example VI

Preparation of magnesium modified ZSM-11

Fourteen grams of the acid form of ZSM-11 was suspended in 33 ml. of a solution containing 75% magnesium nitrate $\cdot$ $6H_2O$ and 25% water, by weight, and allowed to soak at ambient temperature for 4 hours. The liquid was filtered off, and the zeolite placed in an oven to remove residual water by raising the temperature from 30°C to 250°C over a 3 hour period. The catalyst was then placed in a furnace, in air, at 500°C for a period of 3 hours. The weight of the sample, after cooling, was 20.7 grams, which leads to a calculated value of 19.5 weight percent magnesium on the zeolite.

Example VII

Modification of Mg/ZSM-11 with $NO_2$

A sample of the magnesium modified ZSM-11 catalyst of Example VI was treated with nitrogen at 500°C for 15 minutes to remove moisture. The catalyst was cooled to about 25°C and treated with $NO_2$ gas at a rate of 60 cc/min, raising the temperature to 70°C. Heat was applied to increase the temperature to 150°C, including the exothermic heat of reaction. After passage of $NO_2$ for 25 minutes, the catalyst was purged with nitrogen, the temperature was raised to 500°C, and the sample was calcined with air at 100 cc/min for 1 hour before use. Toluene was alkylated with ethylene over the resulting catalyst. Results are summarized in Table V.

TABLE V

Alkylation of toluene with ethylene over Mg ZSM-11 catalyst

| Temperature °C | Untreated catalyst | | Catalyst treated with $NO_2$ | |
|---|---|---|---|---|
| | Conv. % | Para isomer % | Conv. % | Para isomer % |
| 400 | 10.2 | 53.7 | 10.4 | 54.3 |
| 500 | 10.1 | 47.6 | 10.3 | 48.1 |

The Table V data demonstrate that as a result of the treatment with $NO_2$, air increase in toluene conversion and selectivity to the para isomer (ethyltoluene) was observed.

Example VIII

The base catalyst as prepared in Example I is further treated by contact with $H_2S$. Prior to conversion testing, anhydrous hydrogen sulfide is passed over the catalyst in the reactor at 100°C for 15 minutes at the rate of about 50 cc/min/2.2 g of catalyst. This is followed by a brief purge and then by calcination in air for 1 hour at 500°C. Catalyst so treated is then tested for its disproportionation and alkylation performance in the manner described in Example II. Results of such testing are provided in Table VI.

TABLE VI
Para-selectivity of Mg/P and $H_2S$ treated ZSM-5 catalyst

| Reaction | Selectivity to para-isomer (% by weight) | Conversion range (% by weight) |
|---|---|---|
| Toluene disproportionation | | 0.9—13.5 |
| 450°C | 84.1 | |
| 500°C | 79.9 | |
| 550°C | 81.2 | |
| 600°C | 78.9 | |
| Toluene alkylation w/ethylene | | 11.7—9.7 |
| 400°C | 97.6 | |
| 450°C | 96.9 | |
| Toluene alkylation w/methanol | | 6.1—10.2 |
| 400°C | 93.5 | |
| 500°C | 93.1 | |
| 600°C | 91.7 | |

The Table VI data illustrate the increase in para-selectivity provided by treatment of the base catalyst with $H_2S$. Furthermore, it is evident that the decrease in para-selectivity with increased temperature was reduced significantly by $H_2S$ treatment.

Example IX
The base catalyst of Example I was further treated with anhydrous sulfur dioxide, and samples were tested for toluene conversion performance in the manner described in Example II. Catalyst treating conditions and conversion results are shown in Table VII.

9

TABLE VII
Para-selectivity of Mg/P ZSM-5 catalyst
treated with sulfur dioxide

| Sulfur-based treating agent | SO$_2$ | SO$_2$ |
|---|---|---|
| Treating-procedure | 15 min/100°C | 30 min/500°C |
| Run No. | 1 | 2 |
| Tol. disproport. | Selectivity to para isomer, wt % | |
| 450°C | 82.6 | 86.6 |
| 500°C | 82.7 | 87.5 |
| 550°C | 82.4 | 87.2 |
| 600°C | 80.2 | 86.6 |
| Conv. % 0.7—12.4 | 0.8—12.7 | |
| Tol+C$_2$H$_4$ | | |
| 400°C | 97.0 | 98.1 |
| 450°C | 96.1 | 97.9 |
| Conv. % 15:5—12.1 | 14.9—11.6 | |
| Tol+CH$_3$OH | | |
| 400°C | — | 95.3 |
| 500°C | — | 94.0 |
| 600°C | — | 92.0 |
| Conv. % — | 6.6—13.0 | |

The Table VII data illustrate that catalyst treatment with SO$_2$, like treatment with H$_2$S, serves to enhance para-selectivity of Mg/P modified ZSM-5 catalyst for the toluene conversion reactions involved.

Example X
Modification of Mg-ZSM-11 with SO$_2$

A sample of the magnesium modified ZSM-11 catalyst of Example VI was treated with nitrogen at 500°C for 15 minutes to remove moisture. The catalyst was cooled to about 25°C and treated with SO$_2$ gas. An exotherm occurred to raise the temperature and with the aid of a heater, the temperature was increased to about 155°C. After about 25 minutes the SO$_2$ was replaced with a nitrogen purge. The catalyst was then calcined in air flowing through the bed at a rate of 100 cc/min at 500°C.

Toluene was disproportionated to benzene and xylenes over the treated catalyst. Results are summarized in Table VIII.

TABLE VIII
Disproportionation of toluene over Mg-ZSM-11 catalyst

| Temperature °C | Untreated catalyst | | Catalyst treated with SO$_2$ | |
|---|---|---|---|---|
| | Conv. % | Para isomer % | Conv. % | Para isomer % |
| 450 | 0.45 | 32.7 | 0.50 | 33.3 |
| 500 | 1.15 | 30.4 | 1.10 | 31.5 |
| 550 | 2.95 | 29.3 | 3.00 | 29.3 |
| 600 | 6.50 | 26.7 | 7.10 | 27.8 |

The Table VIII data demonstrate that as a result of the treatment with SO$_2$, a desired increase in either toluene conversion or selectivity to the para isomer in the xylene product, or both, were observed.

## Claims

1. A process for enhancing the para-selective properties of zeolite based catalysts useful for the conversion of aromatic compounds to dialkyl-substituted benzene compounds, said catalyst comprising a crystalline zeolite which is free of organic cations and has a silica to alumina ratio of at least 12 and a constraint index from 1 to 12, said process comprising contacting said catalyst with $CO_2$, $NO_2$, $NH_3$, $H_2S$ or $SO_2$ at a temperature of 50°C to 500°C for from 0.1 to 25 hours and then calcining of the catalyst at a temperature of 300°C to 600°C.

2. A process according to claim 1 wherein said zeolite catalyst contains from about 0.25% to 25% by weight of a difficulty reducible oxide.

3. A process according to claim 2 wherein said oxide is magnesium oxide and/or phosphorus oxide.

4. A process according to any preceding claim wherein said zeolite is ZSM-5, ZSM-11, ZSM-12, ZSM-23, ZSM-35, ZSM-38 or ZSM-48.

5. A process according to claim 2 wherein the $CO_2$, $NO_2$, $NH_3$, $H_2S$ or $SO_2$ constitutes from 25% to 100% by volume of a treating medium.

6. A process according to claim 5 wherein said treating medium is passed over said catalyst at a rate of 20 to 100 cc/min/gm catalyst at a temperature of 50°C to 500°C for a period of 0.25 to 3.0 hours.

7. A process according to any preceding claim wherein said treating medium comprises 100% by volume of anhydrous $CO_2$, $NO_2$, $NH_3$, $H_2S$ or $SO_2$.

8. A process according to any preceding claim wherein the catalyst comprises from 1 to 99% by weight of the zeolite material, the balance of said composition comprising a binder for the zeolite.

## Patentansprüche

1. Verfahren zur Verbesserung der Para-Selektivitätseigenschaften von auf Zeolith basierenden Katalysatoren, die für die Umwandlung von aromatischen Verbindungen zu Dialkyl-substituierten Benzolverbindungen wertvoll sind, wobei der Katalysator einen kristallinen Zeolith umfaßt, der frei von organischen Kationen ist und ein Siliciumdioxid/Aluminiumoxid-Verhältnis von mindestens 12 und einen Zwangsindex von 1 bis 12 hat, wobei das Verfahren das Inkontaktbringen des Katalysators mit $CO_2$, $NO_2$, $NH_3$, $H_2S$ oder $SO_2$ bei einer Temperatur von 50 bis 500°C während 0,1 bis 25 Stunden und danach das Kalzinieren des Katalysators bei einer Temperatur von 300 bis 600°C umfaßt.

2. Verfahren nach Anspruch 1, worin der Zeolithkatalysator von etwa 0,25 bis 25 Gew.-% eines schwer reduzierbaren Oxids enthält.

3. Verfahren nach Anspruch 2, worin das Oxid Magnesiumoxid und/oder Phosphoroxid ist.

4. Verfahren nach einem der vorstehenden Ansprüche, worin der Zeolith ZSM-5, ZSM-11, ZSM-12, ZSM-23, ZSM-35, ZSM-38 oder ZSM-48 ist.

5. Verfahren nach Anspruch 2, worin das $CO_2$, $NO_2$, $NH_3$, $H_2S$ oder $SO_2$ von 25 bis 100 Vol.-% des Behandlungsmittels bildet.

6. Verfahren nach Anspruch 5, worin das Behandlungsmittel über den Katalysator bei einer Gewchwindigkeit von 20 bis 100 $cm^3$/min./g des Katalysators bei einer Temperatur von 50 bis 500°C während eines Zeitraumes von 0,25 bis 3,0 Stunden geleitet wird.

7. Verfahren nach einem der vorstehenden Ansprüche, worin das Behandlungsmittel 100 Vol.-% von wasserfreiem $CO_2$, $NO_2$, $NH_3$, $H_2S$ oder $SO_2$ umfaßt.

8. Verfahren nach einem der vorstehenden Ansprüche, worin der Katalysator von 1 bis 99 Gew.-% des Zeolithmaterials umfaßt, wobei der Rest der Zusammensetzung ein Bindemittel für den Zeolith umfaßt.

## Revendications

1. Un procédé pour augmenter les propriétés sélectives en para de catalyseurs à base de zéolite utiles pour la conversion de composés aromatiques en composés benzéniques dialkyle substitués, ce catalyseur comprenant une zéolite cristalline qui est exempte de cations organiques et présente un rapport silice/alumine d'au moins 12 et un indice de contrainte compris entre 1 et 12, ce procédé consistant à mettre ledit catalyseur au contact de $CO_2$, $NO_2$, $NH_3$, $H_2S$ ou $SO_2$ à une température comprise entre 50°C et 500°C pendant 0,1 à 25 heures, puis à calciner pe catalyseur à une température comprise entre 300°C et 600°C.

2. Un procédé selon la revendication 1, dans lequel ledit catalyseur zéolitique contient environ 0,25% à 25% en poids d'un oxyde difficilement réductible.

3. Un procédé selon la revendication 2, dans lequel ledit oxyde est de l'oxyde de magnésium et/ou de l'oxyde de phosphore.

4. Un procédé selon l'une quelconque des revendications précédentes, dans lequel ladite zéolite est la ZSM-5, la ZSM-11, la ZSM-12, la ZSM-23, la ZSM-35, la ZSM-38 ou la ZSM-48.

5. Un procédé selon la revendication 2, dans lequel le $CO_2$, $NO_2$, $NH_3$, $H_2S$ ou $SO_2$ constitue de 25% à 100% en volume de milieu de traitement.

6. Un procédé selon la revendication 5, dans lequel ledit milieu de traitement est envoyé sur ledit catalyseur à un débit de 20 à 100 $cm^3$/min./g de catalyseur, à une température de 50°C à 500°C pendant une période de 0,25 à 3,0 heures.

7. Un procédé selon l'une quelconque des revendications précédentes, dans lequel ledit milieu de traitement comprend 100% en volume de $CO_2$, $NO_2$, $NH_3$, $H_2S$ ou $SO_2$ anhydres.

8. Un procédé selon l'une quelconque des revendications précédentes, dans lequel le catalyseur comprend de 1 à 99% en poids de matière zéolitique, le reste de ladite composition étant formé d'un liant de la zéolite.